(19) **Europäisches Patentamt European Patent Office Office européen des brevets**

(11) **EP 1 589 827 B1**

(12) **EUROPEAN PATENT SPECIFICATION**

(45) Date of publication and mention
of the grant of the patent:
**03.05.2017 Bulletin 2017/18**

(51) Int Cl.:
*A23K 20/174* (2016.01)   *A23K 50/40* (2016.01)
*A61K 31/122* (2006.01)   *A23K 20/179* (2016.01)

(21) Application number: **04708561.8**

(22) Date of filing: **05.02.2004**

(86) International application number:
**PCT/US2004/003220**

(87) International publication number:
**WO 2004/071211 (26.08.2004 Gazette 2004/35)**

(54) **METHODS AND COMPOSITIONS UTILIZING ASTAXANTHIN**

ASTAXANTHIN VERWENDENDE VERFAHREN UND ZUBEREITUNGEN

PROCEDES ET COMPOSITIONS UTILISANT DE L'ASTAXANTHINE

(84) Designated Contracting States:
**AT BE BG CH CY CZ DE DK EE ES FI FR GB GR
HU IE IT LI LU MC NL PT RO SE SI SK TR**

(30) Priority: **05.02.2003 US 445077 P**

(43) Date of publication of application:
**02.11.2005 Bulletin 2005/44**

(73) Proprietor: **IAMS Europe B.V.
7742 PB Coevorden (NL)**

(72) Inventors:
• **CHEW, Boon, Peng
Pullman, Washington 99163 (US)**
• **HAYEK, Michael, Griffin
Dayton, Ohio 45415 (US)**
• **PARK, Jean, Soon
Pullman, Washington 99163 (US)**

(74) Representative: **Zimmermann & Partner
Patentanwälte mbB
Postfach 330 920
80069 München (DE)**

(56) References cited:
WO-A-97/35491          WO-A-98/37874
WO-A-02/079395        WO-A-02/098373
WO-A-03/047528        US-A- 5 679 567
US-A- 5 937 790        US-A- 6 083 520
US-A1- 2003 157 239   US-B1- 6 310 090

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention is directed to compositions which are useful for enhancing immune response, or enhancing longevity in a companion animal, wherein astaxanthin is utilized.

BACKGROUND OF THE INVENTION

In recent years, there has been increasing interest in the health benefits of antioxidants. Antioxidants counteract the effect of harmful free radicals, or reactive oxygen species, that are produced as by-products of normal metabolism. Antioxidant nutrients comprise a variety of compounds having similar ability to neutralize harmful free radicals, and include some commonly known vitamins, such as vitamin A, vitamin C, and vitamin E.

Carotenoids are a subset of antioxidants. Carotenoids are naturally-occurring plant pigments which are absorbed to varying degrees by different species. Common carotenoids include, for example, beta-carotene, lutein, lycopene, astaxanthin, and canthaxanthin. Some antioxidants have also been shown to have anti-oncogenic properties in certain animal species. Beta-carotene, for example, has been found to inhibit human neuroblastoma cell proliferation and canthaxanthin has been shown to prevent chemical-induced carcinogenesis in mice.

Effects on the immune systems of some species of animals have also been associated with certain carotenoids. For example, canthaxanthin increases lymphocyte proliferation in rats and enhances the production of tumor necrosis factor (TNF) by macrophages in hamsters. In a cultured cell assay, astaxanthin and beta-carotene demonstrated the ability to increase *in vitro* antibody response of mouse splenocytes to T-dependent antigens (Jyonouchi *et al.*).

Although much is known about the carotenoids, it is difficult to extrapolate from what is known about the effects of one carotenoid in an animal to determine the effects that another carotenoid may have in the same animal. It is also difficult to determine what the effect of a single carotenoid may be in one animal based upon previous findings in a different type of animal. Carotenoid absorption and metabolism have been determined to be species-specific. Cattle and horses, for example, absorb beta-carotene, while goats and sheep do not absorb carotenoids at all. (Schweigert, F.J., 1998). After a 1931 study demonstrated that a large proportion of beta-carotene provided to cats was not absorbed, many believed that cats were unable to utilize beta-carotene until, Chew *et al.* (2000) determined that domestic cats readily absorb beta-carotene, and Schweigert *et al.* (2002) demonstrated that this absorption is not accompanied by conversion of beta-carotene to vitamin A, indicating that the effects of beta-carotene in cats are not due to its function as a vitamin A precursor.

Even within a single animal, the carotenoids can exhibit differential patterns of absorption. Lutein and zeaxanthin are found concentrated in the human retina, while beta-carotene is generally thought to be absent from retinal tissue due to its inability to cross the blood retinal barrier.

In the field of companion animal nutrition, nutritional components that have a positive effect on the immune function of the animal are desirable. Identifying those nutritional components that enhance immune function in a particular species is a challenge. What is needed are nutritional formulations and methods of use that provide a benefit to a companion animal by enhancing the immune function of the animal.

US 6,310,090 B1 relates to a process and product for enhancing immune response in companion animals using a combination of antioxidants.

WO 97/35491 relates to an agent for increasing the production of/in breeding and production animals.

SUMMARY OF THE INVENTION

The present invention is directed to compositions as defined in the appended claims which are useful for a companion animal, wherein the compositions utilize astaxanthin. The compositions comprise astaxanthin, wherein the composition is adapted for use by a companion animal. Further described are methods selected from the group consisting of attenuating inflammation, enhancing immunity, enhancing longevity, and combinations thereof, comprising administering to a companion animal a composition comprising an effective amount of astaxanthin. The companion animal is, in the preferred embodiment, a domestic dog or cat.

BRIEF DESCRIPTION OF THE DRAWINGS

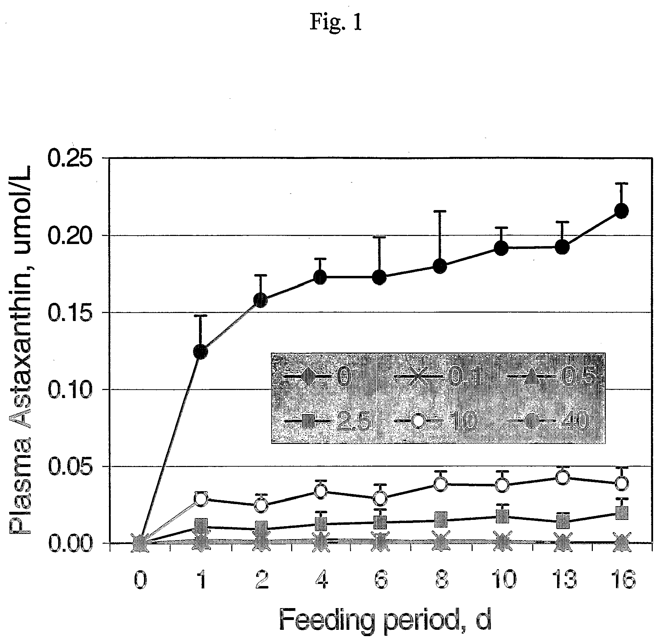

Fig. 1 graphs concentrations of plasma astaxanthin in dogs given daily oral doses of 0, 0.1, 0.5, 2.5, 10 or 40 mg astaxanthin. Values are means $\pm$ SEM (n = 8).

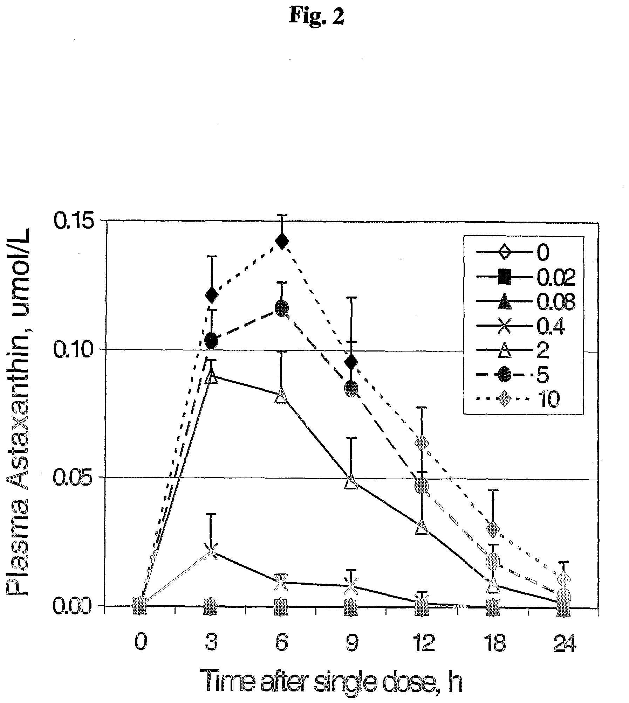

Fig. 2 graphs concentrations of plasma astaxanthin in cats given an oral dose of 0, 0.02, 0.08, 0.4, 2, 5, or 10 mg astaxanthin. Values are means $\pm$ SEM (n = 8).

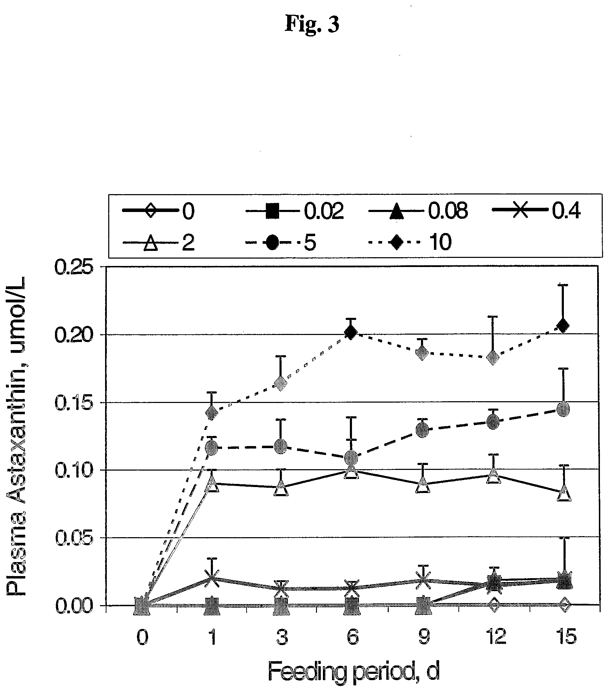

Fig. 3 graphs concentrations of plasma astaxanthin in dogs given daily oral doses of 0, 0.02, 0.08, 0.4, 2, 5, or 10 mg astaxanthin. Values are means $\pm$ SEM (n = 8).

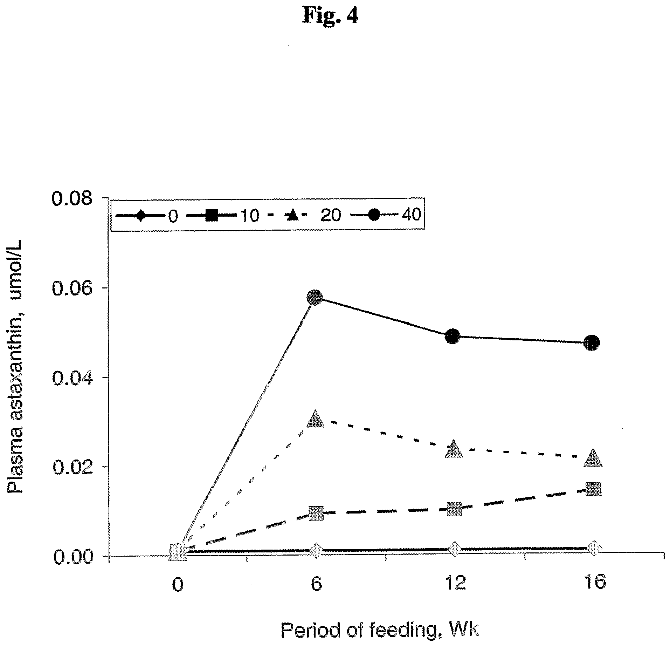

Fig. 4 shows plasma astaxanthin concentrations in dogs fed diets containing 0, 10, 20 or 40 mg astaxanthin for 16 wk. Values are means $\pm$ SEM (n = 14).

Fig. 5 illustrates delayed-type hypersensitivity response (expressed as a percentage of skin thickness measured at 0 h) to an intradermal injection with a polyvalent vaccine in dogs fed 0, 10, 20 or 40 mg astaxanthin daily for 12 (Fig. 5a) or 16 (Fig. 5b) wk. Skin induration was measured at 0, 24, 48 and 72 h post-injection. Values are means $\pm$ SEM (n = 14).

Fig. 6 illustrates percentages (calculated by expressing the number of cells that stained positive for the cell surface marker as a percentage of total number of lymphocytes) of CD21+ B lymphocytes in dogs fed 0, 10, 20 or 40 mg astaxanthin daily for 16 wk. Values are means $\pm$ SEM (n = 14).

Fig. 7 graphs concentrations of plasma IgM (7a) and IgG (7b) in dogs fed 0, 10, 20 or 40 mg astaxanthin daily for 16 wk. All dogs were vaccinated with a polyclonal vaccine on wk 13 and 15. Values are means $\pm$ SEM (n = 14).

Fig. 8 shows natural killer cell cytotoxic activity, expressed as percent kill, in dogs fed 0, 10, 20 or 40 mg astaxanthin daily for 16 wk. Values are means $\pm$ SEM (n = 14).

Fig. 9 illustrates concentrations of plasma C-reactive proteins, expressed in nanograms per milliliter plasma, in dogs fed 0, 10, 20, or 40 mg astaxanthin daily for 16 wk. Values are means $\pm$ SEM (n = 14).

Fig. 10 graphs concentrations of plasma 8-hydroxy-2'-deoxyguanosine (8-OHdG) in dogs fed 0, 10, 20, or 40 mg astaxanthin daily for 16 wk. Values are means $\pm$ SEM (n = 14).

Fig. 11 shows plasma astaxanthin concentrations in cats fed diets containing 0, 1, 5 or 10 mg astaxanthin for 12 wk. Values are means $\pm$ SEM (n = 14).

Fig. 12 illustrates levels of delayed-type hypersensitivity response (expressed as a percentage of skin thickness measured at 0 h) to an intradermal injection with a polyvalent vaccine in cats fed 0, 1, 5 or 10 mg astaxanthin daily for 12 wk. Skin induration was measured at 0, 24, 48 and 72 h post-injection. Values are means $\pm$ SEM (n = 14).

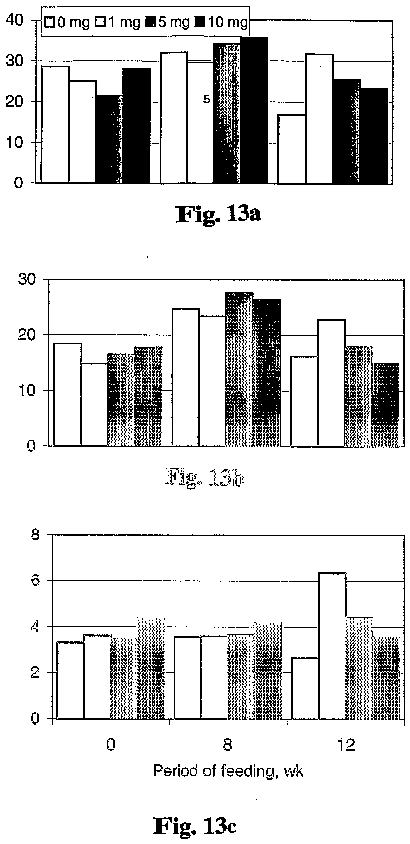

Fig. 13 illustrates incorporation of [$^3$H]-thymidine by con A- (2.5 $\mu$g/mL), PHA- (1.25 $\mu$g/mL) or PWM- (2.5 $\mu$g/mL) induced PBMC proliferation in cats fed 0, 1, 5, or 10 mg astaxanthin daily for 12 wk. Values are means $\pm$ SEM (n = 14).

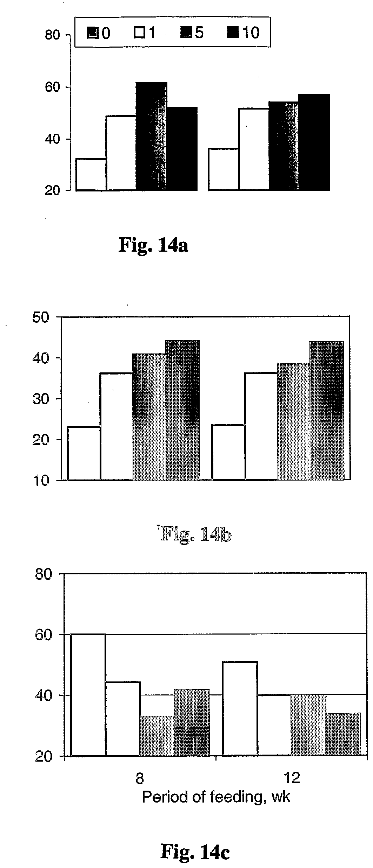

Fig. 14 illustrates percentages (calculated by expressing the number of cells that stained positive for the cell surface marker as a percentage of total number of lymphocytes) of CD5+ total T cells (Fig. 14a), CD4+ Th cells (Fig. 14b) and CD21+ B cells (Fig. 14c) in cats fed 0, 1, 5 or 10 mg astaxanthin daily for 12 wk. Values are means $\pm$ SEM (n = 14).

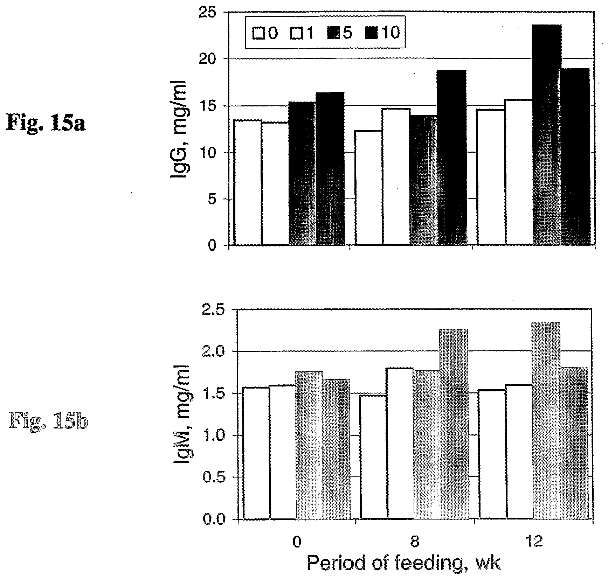

Fig. 15 shows concentrations of plasma IgG (Fig.15a) and IgM (Fig.15b) in cats fed 0, 1, 5 or 10 mg astaxanthin daily for 12 wk. All cats were vaccinated with a polyclonal vaccine on wk 9 and 10. Values are means $\pm$ SEM (n = 14).

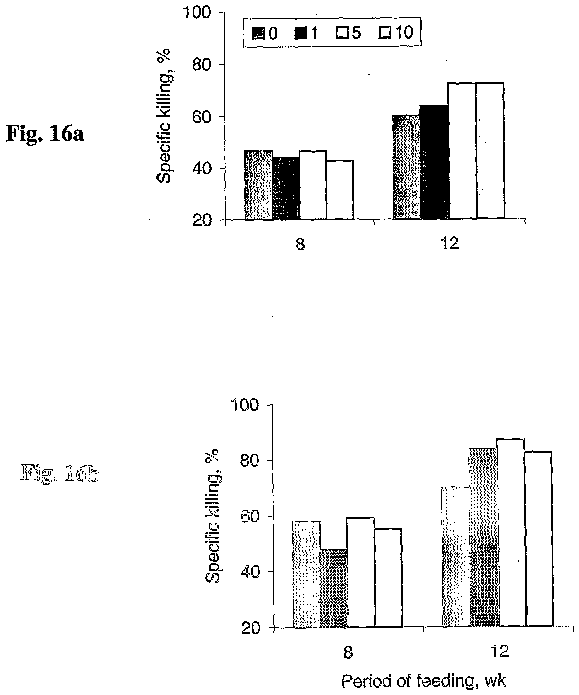

Fig. 16 graphs natural killer cell cytotoxic activity in cats fed 0, 1, 5 or 10 mg astaxanthin daily for 12 wk. Values are means $\pm$ SEM (n = 14).

## DETAILED DESCRIPTION OF THE INVENTION

Various documents including, for example, publications and patents, are recited throughout this disclosure. The citation of any given document is not to be construed as an admission that it is prior art with respect to the present invention.

All percentages and ratios are calculated by weight unless otherwise indicated. All percentages and ratios are calculated based on the total composition unless otherwise indicated.

Referenced herein are trade names for components including various ingredients utilized in the present invention. The inventors herein do not intend to be limited by materials under a certain trade name. Equivalent materials (*e.g.*, those obtained from a different source under a different name or reference number) to those referenced by trade name may be substituted and utilized in the descriptions herein.

In the description of the invention various embodiments or individual features are disclosed. As will be apparent to the ordinarily skilled practitioner, all combinations of such embodiments and features are possible and can result in preferred executions of the present invention.

The compositions herein may comprise, consist essentially of, or consist of any of the features or embodiments as described herein.

While various embodiments and individual features of the present invention have been illustrated and described, various other changes and modifications can be made without departing from the scope of the invention. As will also be apparent, all combinations of the embodiments and features taught in the foregoing disclosure are possible and can result in preferred executions of the invention.

Composition of the Present Invention

The compositions herein are adapted for use by a companion animal. As used herein, the term "companion animal" means a domestic animal preferably including (for example) dogs, cats, horses, pigs (e.g., pot-bellied pigs), rabbits, and the like. Domestic dogs and cats are particularly preferred. In this respect, as will be well-understood by the ordinarily skilled artisan, the primary use of the compositions described herein is for companion animal use and the compositions are therefore formulated as such.

Astaxanthin (3,3'-dihydroxy-beta, beta-carotene-4,4'-dione), an oxycarotenoid or alhpa-hydroxy-ketocarotenoid, is a potent antioxidant (Martin *et al.,* 1999). It is commonly used in aquaculture and in the poultry industry as a feed additive, primarily due to its red pigment. The antioxidant activity of astaxanthin against certain reactive oxygen species has been observed to be higher than that of beta-carotene, canthaxanthin, lutein, alpha-tocopherol, tunaxanthin, and zeaxanthin (Naguib, 2000; Miki, 1991). Rainbow trout fed astaxanthin-rich yeast have demonstrated increased ability to reduce oxidized oil-induced oxidative stress (Nakano *et al.,* 1999) and lower levels of serum lipid peroxides and transaminase activities (Nakano *et* al., 1995 and Nakano *et al.,* 1996). Whether directly or indirectly related to its antioxidant activity, astaxanthin enhanced both humoral (Jyonouchi *et al.,* 1995) and cell-mediated (Chew *et al.,* 1999b) immune responses, and inhibited mammary (Chew *et al.,* 1999a) and bladder (Tanaka *et al.,* 1994) tumor growth in rodents. It has also been shown to enhance mitogen-induced splenocyte proliferation (Chew *et al.,* 1999) in mice. In *Helicobacter pylori*-infected mice fed astaxanthin-rich algae extract, the bacterial load and gastric inflammation were reduced, apparently due to a shift in T-lymphocyte from a Th1 response dominated by IFN-gamma to a mixed Th1/Th2 response with IFN-gamma and IL-4 (Bennedsen *et al.,* 1999).

Until now, however, it has not been known whether companion animals (*e.g.*, dogs and cats) could absorb and utilize astaxanthin in effective pharmacological amounts, nor have the effects of any such absorption in cats or dogs been determined. In studies performed by the inventors, domestic dogs and cats which are fed astaxanthin show significant uptake by the blood and by all sub-cellular organelles of blood leukocytes. The present invention, therefore, provides compositions useful for administering a companion animal a composition which contains astaxanthin as an ingredient or a food additive in an amount sufficient to provide, for example, from about 0.001 mg to about 40 mg, daily, of astaxanthin. Such a diet provides sufficient astaxanthin to be absorbed by the animal and supplied to the blood, *e.g.*, plasma, blood leukocytes, in the animal.

The inventors have discovered that both domestic dogs and cats are able to absorb dietary astaxanthin.

Furthermore, the inventors have discovered that circulating astaxanthin is significantly absorbed by blood leukocytes and is associated with high-density lipoprotein (HDL) in such animals. They have also discovered that astaxanthin is also distributed in the various sub-cellular organelles. Such absorption of astaxanthin into the various organelles of leukocytes is believed to (1) protect these cells from oxygen free radical attack and/or (2) directly regulate nuclear events. Thus, feeding companion animals such as dogs and cats a composition containing effective amounts of astaxanthin provides astaxanthin at important cellular sites in the body tissues of the animal that results in an up-regulation of immune function and improved health in these animals.

Astaxanthin can be provided as free astaxanthin or as astaxanthin diester. Naturally produced astaxanthin can be obtained from fungi, crustaceans, and algae, e.g., *Haematococcus* sp. (*e.g.*, as described in U.S. Patent No. 5,744,502). Astaxanthin is also produced by wild-type and genetically engineered *Pfaffia* yeast, and is commercially available from Archer Daniels Midland Co.; Aquasearch Inc.; AstaCarotene AB; Cyanotech Corporation and Micro Gaia, Inc. Synthetically produced astaxanthin is also commercially available from Hoffman-LaRoche, Ltd. The form of astaxanthin administered can be chosen to provide a more bioavailable product, for example, administration as a beadlet, oleoresin, or the like.

The compositions used herein are, in a preferred embodiment, pet food compositions. As used herein, the term "pet food composition" means a composition that is intended for ingestion by a companion animal. These will advantageously include foods intended to supply necessary dietary requirements, as well as treats (*e.g.*, dog biscuits) or other food supplements. Optionally, the composition herein may be a pet food composition such as a dry composition (for example, kibble), semi-moist composition, wet composition, or any mixture thereof. Alternatively or additionally, the composition is a supplement, such as a gravy, drinking water, yogurt, powder, suspension, chew, treat (*e.g.*, biscuits) or any other delivery form. As an example, the astaxanthin may be blended with the other components of the composition to provide the beneficial amounts needed, or may be added to the composition prior to offering it to the animal, for example, using a sprinkled powder.

As an example, in one embodiment the composition is nutritionally balanced. As used herein, the term "nutritionally balanced," with reference to the companion animal composition, means that the composition has known required nutrients to sustain life in proper amounts and proportion based on recommendations of recognized authorities in the field of companion animal nutrition. Nutritionally balanced compositions are widely known and widely used in the art.

The compositions used herein may optionally comprise one or more further components. Other components are beneficial for inclusion in the compositions used herein, but are optional for purposes of the invention. In one embodiment, the food compositions may comprise, on a dry matter basis, from about 20% to about 50% crude protein, alternatively from about 20% to about 40% crude protein, by weight of the food composition, or alternatively from about 20% to about 35% crude protein. The crude protein material may comprise vegetable proteins such as soybean, cottonseed, and peanut, or animal proteins such as casein, albumin, and meat protein. Non-limiting examples of meat protein useful herein include a protein source selected from the group consisting of beef, pork, lamb, poultry, fish, vegetable, and mixtures thereof.

The compositions may comprise, on a dry matter basis, from about 5% to about 40% fat, alternatively from about 10% to about 35% fat, by weight of the food composition.

The compositions of the present invention may further comprise a source of carbohydrate. Grains or cereals such as rice, corn, milo, sorghum, barley, wheat, and the like are illustrative sources.

The compositions may also contain other materials such as dried whey and other dairy by products.

The compositions may also comprise at least one fiber source for improved gastrointestinal health. Such fiber sources may comprise, for example, at least one moderately fermentable fiber. Moderately fermentable fiber has previously been described to provide a benefit to the immune system of a companion animal. Moderately fermentable fiber or other compositions known to those of skill in the art which provide a prebiotic composition to enhance the growth of probiotic microorganisms within the intestine may also be incorporated into the composition to aid in the enhancement of the benefit provided by the present invention to the immune system of an animal. Additionally, probiotic microorganisms, such as *Lactobacillus* or *Bifidobacterium* species, for example, may be added to the composition.

Given the disclosure of the present invention, an appropriate amount of astaxanthin may be determined by those of skill in the art, taking into account the type of composition (*e.g.*, nutritionally balanced pet food composition *versus* supplement), the average consumption of specific types of compositions by different animals, and the manufacturing conditions under which the food is prepared. As an example, the compositions may, in certain embodiments, comprise less than about 3% of astaxanthin, by weight of the composition. In an even further embodiment, the compositions may comprise from about 0.0001% to about 2%, or from about 0.001% to about 1%, or from about 0.001% to about 0.5%, of astaxanthin, all by weight of the composition.

Methods

The methods described herein comprise orally administering (*i.e.*, through ingestion) a composition of the present invention to a companion animal and most preferably a domestic dog or cat, to attenuate inflammation, enhance immune response, enhance longevity, or combinations thereof.

Described is also a method of providing astaxanthin in a dosage effective to enhance the immune response of a dog or cat wherein the astaxanthin is associated with high density lipoprotein (HDL). The method also provides a sufficient amount of astaxanthin in the diet of the animal so that astaxanthin is absorbed by the leukocytes of the animal.

The method provides astaxanthin at an effective level to increase the cell-mediated immune response in a dog or cat. The method also provides an effective level of astaxanthin to increase the humoral immune response in a companion animal, as well as *in vivo* production of IgG and IgM.

By providing improved immune function to the animal, also provided is a method for promoting longevity in a companion animal, comprising the step of feeding the animal a diet comprising an effective amount of astaxanthin for a time sufficient for the astaxanthin to enhance the animal's ability to elicit an immune response.

The compositions of the present invention are ingested by companion animals in need of (for example) enhanced immune response, a palatable food source, or means to satisfy hunger or nutritional needs. The compositions may also be ingested as a supplement to normal dietetic requirements.

As used herein, the term "orally administering" with respect to the companion animal means that the animal ingests or a human is directed to feed, or does feed, the animal one or more compositions herein. Preferably, the composition is a pet food composition or a supplement, as has been described herein. Wherein the human is directed to feed the composition, such direction may be that which instructs and or informs the human that use of the composition may and / or will provide the referenced benefit, for example, enhanced immune response. For example, such direction may be oral direction (*e.g.*, through oral instruction from, for example, a physician, veterinarian, or other health professional, or radio or television media (*i.e.*, advertisement), or written direction (*e.g.*, through written direction from, for example, a physician, veterinarian, or other health professional (*e.g.*, scripts), sales professional or organization (*e.g.*, through, for example, marketing brochures, pamphlets, or other instructive paraphernalia), written media (*e.g.*, internet, electronic mail, or other computer-related media), and / or packaging associated with the composition (*e.g.*, a label present on a container holding the composition). As used herein, "written" means through words, pictures, symbols, and / or other visible descriptors. Such information need not utilize the actual words used herein, for example, "enhance", "immune", "response", or the like, but rather use of words, pictures, symbols, and the like conveying the same or similar meaning are contemplated within the scope of this invention.

The compositions described herein may be used as a supplement to ordinary dietetic requirements or may serve as the primary food for the companion animal (and, as such, the supplements or foods may be nutritionally balanced). Administration may be on as as-needed or as-desired basis, for example, once-monthly, once-weekly, or daily (including multiple times daily). When utilized as a supplement to ordinary dietetic requirements, the composition may be administered directly to the mammal or otherwise contacted with or admixed with daily feed or food. When utilized as a daily feed or food, administration will be well-known to those of ordinary skill.

The amount of composition utilized may be dependent on a variety of factors, including the condition and/or age of the companion animal, the quality of the pet food composition or supplement (where applicable), and size or breed of the companion animal (where applicable). As guidance, a companion animal may be administered from about 0.001 mg to about 40 mg, daily, of astaxanthin. As a further example, cats may be administered from about 0.02 mg to about 10 mg, daily, of astaxanthin. As a further example, dogs may be administered from about 1 mg to about 40 mg, daily, of astaxanthin.

Furthermore, plasma concentration of astaxanthin may be increased in a cat to from about 20 nmol/L to about 0.14 $\mu$mol/L after administering the composition. Plasma concentration of astaxanthin in a dog may be increased to from about 0.01 $\mu$mol/L to about 0.14 $\mu$mol/L after administering the composition.

The invention is illustrated by the following non-limiting examples:

**Example 1**

**Effects of Astaxanthin Supplementation in Dogs**

**Materials and Methods**

Female Beagle dogs (9 to 10 mo old; 8.2 $\pm$ 0.2 kg body weight; Covance Research Products Inc., Kalamazoo, MI) were randomly assigned (n = 14/treatment) to be fed 0, 10, 20, or 40 mg astaxanthin (109 g astaxanthin/kg oleoresin concentrate from *Haematococcus pluvialis*, astraZanthin™, La Haye Laboratories, Redmond, WA) daily for 16 weeks. Astaxanthin was incorporated into a commercial basal diet (The Iams Co., Lewisburg, OH) and fed two times daily (200 g food/d). The diet composition was as follows (g/kg): 85.3 moisture, 275.8 protein, 60.7 ash, 115.0 fat, 9.9 Ca, 9.3 P, 21.3 crude fiber, and 18,914 kJ/kg gross energy; the n-6:n-3 fatty acid ratio was 7.9. Dogs were housed in 2 x 3 m pens (2 dogs/pen) in a temperature (20 to 22°C) and light- (14 hour light) controlled facility. Body weight was recorded on week 0, 4, 8, and 16. Blood was collected on wk 0, 6 and 12 for assessing immune function. All dogs were vaccinated (Vanguard 5™, Smithkline Beacham, West Chester, PA) twice on week 12 and 14 and blood again taken on week 16 to assess post-vaccination immune responses.

*Delayed-type hypersensitivity*. On week 12 (before vaccination) and 16 (after vaccination), all dogs were injected intradermally with 100 microliters of (1) saline (8.5 g/L; negative control), (2) an attenuated polyvalent vaccine containing canine distemper virus, adenovirus type-2, parainfluenza virus and parvovirus (undiluted; Vanguard 5™, Smithkline Beacham, West Chester, PA), and (3) phytohemagglutinin (PHA, 0.5 g/L) as previously described (Chew *et al.*, 2000) to assess cutaneous delayed-type hypersensitivity (DTH). Skin induration was measured at 0, 24, 48 and 72 hours post-injection.

*Hematology*. Complete blood count (white blood cell, RBC and platelet counts, lymphocyte, monocyte and granulocyte differential counts, hematocrit, hemoglobin, and mean corpuscular volume, hemoglobin and hemoglobin concentration, and platelet volume) was performed on a hematology analyzer (Vet ABC-Hematology Analyzer, Heska, Fort Collins, CO) using EDTA-treated blood.

*Lymphoproliferation.* The proliferation response of peripheral blood mononuclear cell (PBMC) to PHA (2 and 10 mg/L final concentration), concanavalin A (Con A; 1 and 5 mg/L), and pokeweed mitogen (PWM; 0.25 and 1.25 mg/L) was assessed on week 0, 6, 12 and 16 using whole blood cultures (Chew et al., 2000). Whole blood was cultured in order to mimic *in vivo* conditions.

*Leukocyte subset.* Subpopulations of CD3 (total T), CD4 (Th), CD8 (Tc), MHC II (activated lymphocytes), and CD21 (mature B cells) were quantitated by flow cytometry on week 0, 6, 12 and 16 (Chew et al., 2000).

*IgG and IgM.* Concentrations of IgG (sheep anti-dog IgG; sensitivity = 16 micrograms/L) and IgM (goat anti-dog IgM; sensitivity = 31 micrograms/L) in plasma were analyzed by ELISA using a commercial kit (Bethyl Lab., Inc., Montgomery, Tx).

*Natural killer cell cytotoxic activity.* Canine thyroid adenocarcinoma cells (target cells) were resuspended to $2 \times 10^5$ cells/mL in Dulbecco's Modified Eagles Medium (Sigma, St. Louis, MO) containing 100 mL/L fetal bovine serum, 100 U/mL penicillin, and 100 g/L streptomycin sulfate. Ficol-separated PBMC (effector cells) were resuspended to $1 \times 10^6$ and $2 \times 10^6$ cells/mL and 100 microliters added to the target cells in 96-well flat-bottom plates to provide effector:target ratios of 5:1 and 10:1. After incubating for 8 h, 20 microliters (5 g/L) of MTT were added and incubated for 4 hours. The supernatant was removed and the formazan resuspended in 100 microliters isopropanol. Optical density was measured at 550 nm and the percent of specific cytotoxicity calculated as follows:

$$\% \text{ Specific cytotoxicity} = 1 - (OD_{effector+target} - OD_{effector})/OD_{target} \times 100.$$

*C-Reactive protein.* Changes in acute phase proteins were assessed in plasma by measuring the C-reactive protein (CRP) concentrations using horseradish peroxidase-labeled anti-canine CRP in a solid-phase sandwich immunoassay (Tri Delta Diagnostics, Morris Plains, NJ).

*Oxidative damage to DNA.* 8-Hydroxy-2'-deoxyguanosine (8-OHdG) was measured in plasma ELISA (BIOXYTECH™ 8-OHhdG-EIA Kit, OxisResearch, Portland, OR; sensitivity = 0.5 micrograms/L).

## Results

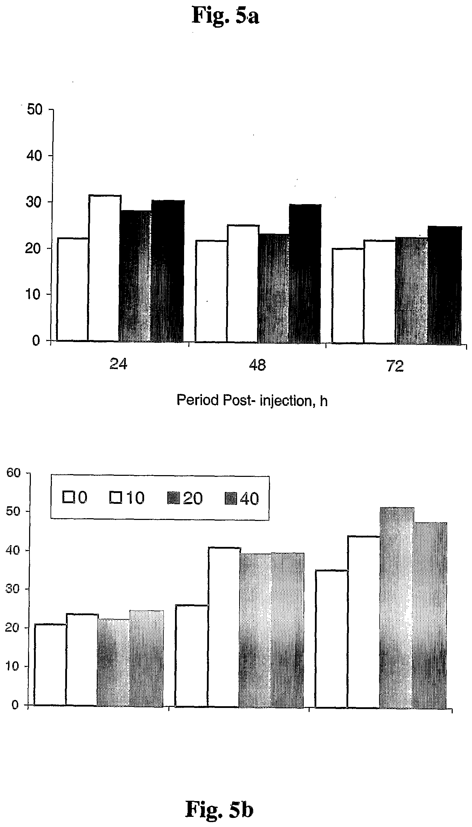

Diet did not significantly influence body weight throughout the study. Body weight averaged $8.18 \pm 0.16$ and $8.57 \pm 0.11$ kg on wk 0 and 16, respectively. Astaxanthin was not detectable in the plasma of all dogs prior to supplementation and in un-supplemented dogs during the study. However, astaxanthin increased in a dose-dependent manner through week 16 (Figure 5). Maximal blood concentrations were observed by wk 6 in all supplemented dogs.

*Delayed-type hypersensitivity.* Diet did not significantly influence skin induration response to saline, PHA or vaccine on wk 0 and 8. However, dogs fed 10, 20 and 40 mg astaxanthin had higher DTH response at 48 and 72 h after an intradermal challenge with the vaccine on wk 12 (Figure 5). This response was observed earlier (at 24 and 48 h) on wk 16 after dogs were vaccinated on wk 12 and 14. No similar response was observed with PHA.

*Hematology.* Blood hematology generally showed no dietary effects throughout the study.

*Lymphoproliferation.* Dogs fed 20 mg astaxanthin had higher con A-induced PBMC proliferation than un-supplemented dogs on week 12. However, astaxanthin did not influence changes in PHA- or PWM-stimulated PBMC proliferation at all time periods.

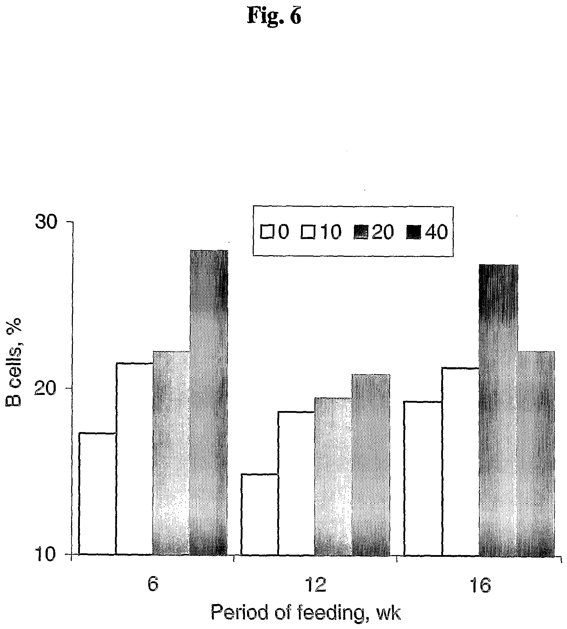

*Leukocyte subpopulations.* Dietary astaxanthin generally increased the population of B cells at week 6 and 12 in a does-dependent manner (Figure 6). Post-vaccination on week 16, dogs fed 20 mg astaxanthin showed the highest response (Figure 6). Astaxanthin did not effect changes in the CD4, CD8, and MHC class II populations at any time periods studied. However, dogs fed 40 mg astaxanthin showed a transient decrease in the CD3 population.

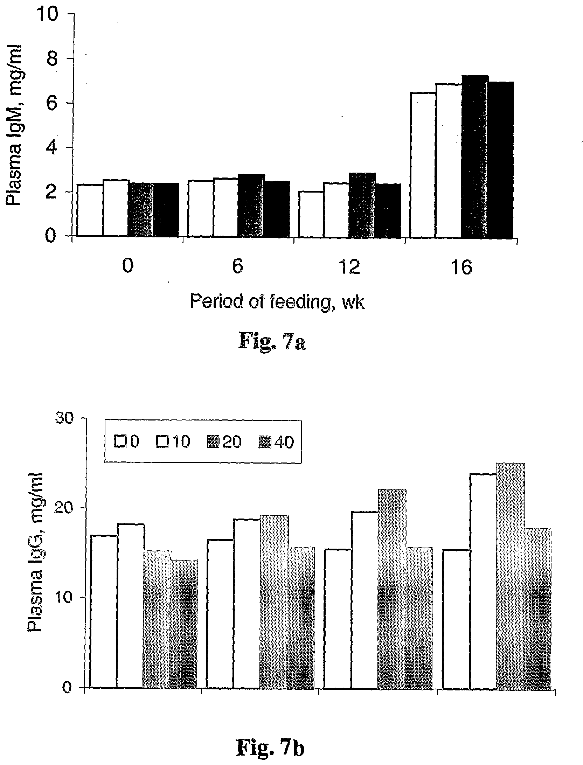

*Immunoglobulin production.* Concentrations of plasma IgG was higher on week 12 in dogs fed 20 mg astaxanthin and again post-vaccination on week 16 (Figure 7). Higher (40 mg) dietary astaxanthin did not produce the same stimulation on IgG production. Plasma IgM concentration was not influenced by dietary astaxanthin pre-vaccination (week 0 to 12) (Figure 7). Vaccination on week 12 and 14 generally increased plasma IgM concentrations approximately 300% in all dogs. Dogs fed 20 mg astaxanthin showed significantly higher IgM concentrations on week 16. At the commencement of the study, concentrations of IgG and IgM were similar among treatment groups.

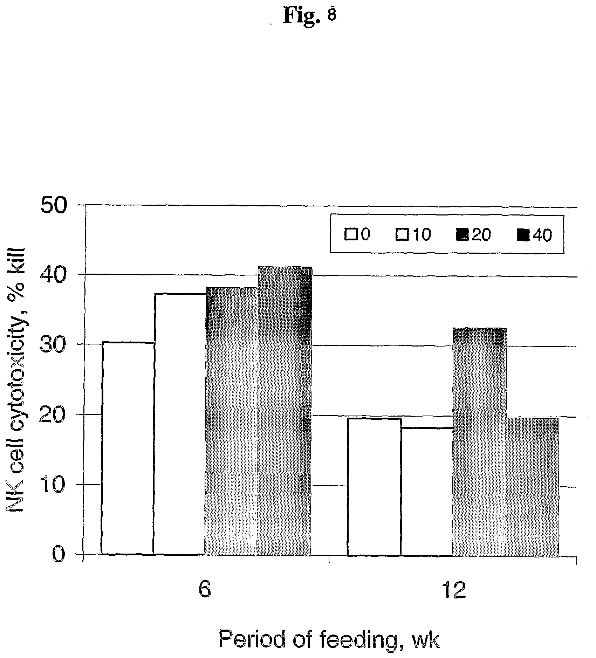

*Natural killer cell cytotoxic activity.* Dietary astaxanthin produced a dose-dependent increase in NK cell activity on week 6; dogs fed 40 mg astaxanthin being significantly higher than control (Figure 8). On week 12, dogs fed 20 mg but not those fed 40 mg astaxanthin had higher NK cell cytotoxic activity than control. This same trend continued through week 16. No treatment difference was observed on week 0.

*C-Reactive protein.* Concentrations of plasma CRP were similar through week 12 of the study (averaged 4.48 mg/L). However, dietary astaxanthin decreased plasma C-reactive protein concentrations post-vaccination on week 16.

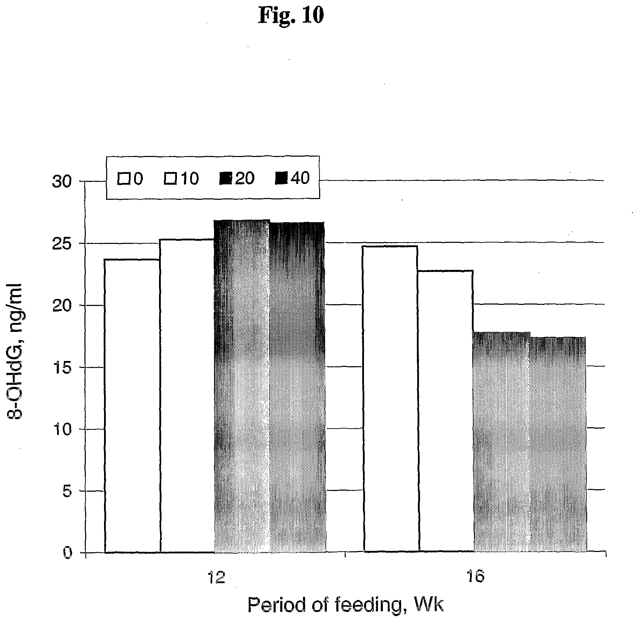

*DNA Oxidative damage.* There was no dietary influence on the concentration of 8-OHdG on wk 12 (Figure 10). As with C-reactive protein, dietary astaxanthin inhibited the production of 8-OHdG in the plasma of supplemented dogs. There was no further decrease in plasma 8-OHdG concentrations in dogs fed 40 mg astaxanthin.

Dietary astaxanthin enhanced both cell-mediated and humoral immune responses in dogs. Daily doses 10 to 40 mg astaxanthin increased delayed-type hypersensitivity (DTH) response to a specific antigen (the vaccine) but not to PHA, a nonspecific antigen, by week 12 of feeding. Post-vaccination, DTH response was similarly enhanced with dietary astaxanthin. However, the overall skin induration response was more rapid post-vaccination (observed 24 hour after intradermal challenge) than before vaccination (48 hour after intradermal challenge). A heightened DTH response from feeding astaxanthin in dogs also was observed over that of other carotenoids previously investigated, including β-carotene (Chew *et al.* 2000), and lutein (Kim *et al.* 2000) as determined by the cutaneous response, which is generally considered a reliable clinical method for assessment of *in vivo* T-cell function in dogs (Miyamoto *et al.* 1995).

The heightened DTH response is consistent with a higher lymphocyte proliferative response to con A, a T-cell mitogen. The highest blastogenic response was observed in this study with 20 mg astaxanthin. Natural killer (NK) cells serve as an immuno-surveillance system against tumors, and dietary astaxanthin also demonstrated the ability to enhance NK cell activity.

Dietary astaxanthin also stimulated humoral immunity, increasing IgG and IgM production over un-supplemented controls after vaccination, with an optimum response in these studies observed for 20mg/d astaxanthin supplementation. In this study, the heightened antibody response paralleled the increase in the B cell subpopulation. In fact, on week 16, dogs fed 20 mg had both the highest B cell subpopulation and the highest concentrations of IgG and IgG. This is compatible with previous studies done in dogs with 20 mg β-carotene (Chew *et al.,* 2000) and 20 mg lutein (Kim *et al.,* 2000) where those dogs also exhibited higher concentrations of plasma IgG than un-supplemented animals.

Astaxanthin-supplemented canines also exhibited lower concentrations of circulating CRP after vaccination challenge. Blood CRP concentrations increase in response to infection, inflammation and other disease states involving tissue necrosis, all of which may be indicative of high oxidative stress.

### Example 2

### Effects of Astaxanthin Supplementation in Cats

### Materials and Methods

Female domestic short hair cats (8 to 9 months old; 3.2 ± 0.04 kg body weight; Liberty Farms, Waverly, NY) were randomly assigned (n = 14/diet) to be fed 0, 1, 5, or 10 mg astaxanthin (109 g astaxanthin/kg oleoresin concentrate from *Haematococcus pluvialis*, astraZanthin™, La Haye Laboratories, Redmond, WA) daily (based on an average intake of 90 g food/day) for 12 weeks. Astaxanthin was incorporated into a commercial basal diet (The Iams Co., Lewisburg, OH) and fed *ad libitum.* The diet composition was as follows (g/kg): 63.1 moisture, 350.6 protein, 62.7 ash, 213.6 fat, 10.0 Ca, 7.6 P, and 7.1 crude fiber, 21,707 kJ/kg gross energy; the n-6:n-3 fatty acid ratio was 9.9. Cats were housed in a temperature (20 to 22°C) and light-controlled (14 hour light) facility and body weights were determined at weeks 0, 4, 8 and 12. Blood was collected at week 0 and 8 to assess immune function. To assess the dietary effects of astaxanthin on immune response after an antigenic challenge, all cats were vaccinated (Felocell™, Pfizer, New York) two times (weeks 8 and 10) and blood was collected at week 12 to measure the same immune parameters.

*Delayed-type hypersensitivity.* Cutaneous delayed-type hypersensitivity (DTH) response was assessed at weeks 0, 8 (pre-vaccination), and 12 (post-vaccination), as described by Kim *et al.,* 2000b. Cats were injected intradermally with 100 μl of either (1) saline (8.5 g/L; negative control), or (2) an attenuated polyvalent vaccine containing feline herpesvirus-1, calicivirus, parvovirus and *Chlamydia psittaci* (Felocell™, Pfizer, NY, NY) to measure specific immunity, and (3) concanavalin A (con A, 0.5 g/L). Skin induration was measured at 0, 24, 48 and 72 hours post-injection.

*Hematology.* Hematology parameters (white blood cell, RBC and platelet counts, lymphocyte, monocyte and granulocyte differential counts, hematocrit, hemoglobin, and mean corpuscular volume, hemoglobin and hemoglobin concentration, and platelet volume) were measured using a hematology analyzer (Vet ABC-Hematology Analyzer, Heska, Fort Collins, CO).

*Lymphoproliferation.* Mitogen-induced peripheral blood mononuclear cell (PBMC) proliferation was assessed by incubating whole blood in the presence of PHA (0.25 and 1.25 mg/L final concentration), con A (0.5 and 2.5 mg/L), and pokeweed mitogen (PWM; 0.025 and 0.125 mg/L) as previously described (Chew *et al.*, 2000). Whole blood was used in order to simulate *in vivo* conditions. Results are reported as stimulation index (cpm of mitogen-stimulated ÷ cpm of unstimulated cultures).

*Leukocyte subset.* Blood collected at week 0, 8 and 12 was phenotyped for populations of CD3 (total T), CD4 (Th), CD8 (Tc), MHC II (activated lymphocytes), and CD21 (mature B cells) by flow cytometry (FACSCalibur, Becton Dickenson, San Jose, CA) as previously described (Chew *et al.*, 2000).

*IgG and IgM.* Plasma concentrations of IgG (sheep anti- IgG; sensitivity = 16 μg/L) and IgM (goat anti- IgM; sensitivity = 31 μg/L) were analyzed by ELISA using a commercial kit (Bethyl Lab., Inc., Montgomery, TX).

*Natural killer cell cytotoxic activity.* Cultures of Crandell feline kidney fibroblast cells (CrFK, ATCC CRL-9761; Crandell et al., 1973) were grown in Dulbecco's Modified Eagles Medium (Sigma, St. Louis, MO.) with 10 % fetal bovine serum, 100 U/mL penicillin, and 100 g/L streptomycin sulfate. At 70-90 % confluence, cells were trypsinized, washed and adjusted to $2 \times 10^5$ cells/mL. One hundred microliters of cell suspension was pipeted into each well of 96-well flat bottom plates (Nunclon, Denmark) and incubated at 37°C for 8 hours. Ficoll-separated PBMC were adjusted to $1 \times 10^6$/mL or $2 \times 10^6$/mL and 100 $\mu$L of the cell suspensions were added to the CrFK target cells to provide effector:target cell ratios of 5:1 and 10:1. After incubating for 8 hours, 20 $\mu$L MTT (5 g/L) were added and the mixture incubated for 4 hours. The supernatant was removed and the formazan resuspended in 100 $\mu$L isopropanol. Optical density was measured at 550 nm and the percent of specific cytotoxicity calculated as follows:

$$\% \text{ Specific cytotoxicity} = 1 - (\text{OD}_{\text{effector+target}} - \text{OD}_{\text{effector}})/\text{OD}_{\text{target}} \times 100.$$

**Results**

Body weight averaged $3.23 \pm 0.04$ and $3.22 \pm 0.06$ kg at weeks 0 and 12, respectively and was not significantly different between treatment. While astaxanthin was not detectable in the plasma of un-supplemented cats, there was generally a dose-dependent increase in astaxanthin concentrations (Figure 7). After a rapid initial increase in plasma astaxanthin concentrations at week 8, plasma astaxanthin continued to increase, albeit more gradually, through week 12.

*Delayed-type hypersensitivity.* Dietary astaxanthin stimulated DTH response to both the specific (vaccine) and nonspecific (con A) antigenic challenge (Figure 8) but not to saline (not shown) at wk 8. Maximal skin induration response to vaccine was observed at 72 h post-injection and significant enhancement was observed in cats fed 10 mg astaxanthin. Cats fed 5 or 10 mg astaxanthin had higher DTH response to con A at 24 and 48 h after an intradermal challenge (Figure 8). The DTH response with dietary astaxanthin was somewhat diminished after vaccination on wk 12. Cats fed 10 mg astaxanthin still showed enhanced DTH response to vaccine, but at 24 h after injection (Figure 8). Response to con A was significant only with 1 mg astaxanthin.

*Hematology.* Dietary astaxanthin generally did not influence blood hematology parameters (white blood cell, RBC and platelet counts, lymphocyte, monocyte and granulocyte differential counts, hematocrit, hemoglobin, and mean corpuscular volume, hemoglobin and hemoglobin concentration, and platelet volume); all values were within the normal range.

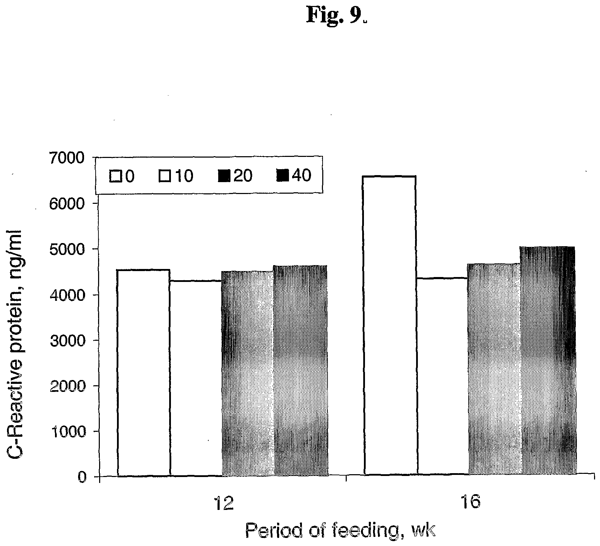

*Lymphoproliferation.* Even though dietary astaxanthin did not influence mitogen- induced PBMC proliferation by week 8, cats fed 1 mg but not those fed higher amounts of astaxanthin showed higher proliferative response to ConA, PHA, and PWM post- vaccination on week 12 (Figure 9).

*Leukocyte subpopulations.* Dietary astaxanthin increased the populations of CD5+ total T cells and CD4+ T helper (Th) cells by weeks 8 and 12 (Figure 10). Increases were generally dose-dependent. In contrast, astaxanthin decreased the population of B cells. Diet did not influence distribution of CD8+ T cytotoxic (Tc) cells (averaged $(4.7 \pm 0.4)$ nor MHC class II (averaged $94.1 \pm 1.0$) populations.

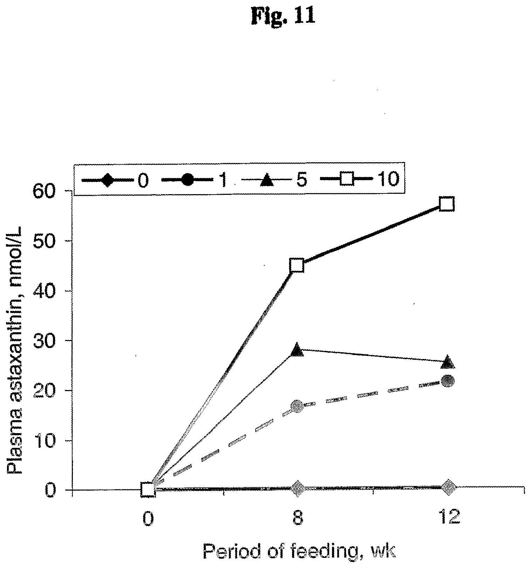

*Immunoglobulin production.* Concentrations of plasma IgG and IgM were higher on week 8 in cats fed 10 mg astaxanthin (Figure 11). Even concentrations of both IgG and IgM were still numerically higher in these cats post-vaccination on week 12, only those fed 5 mg astaxanthin were significant.

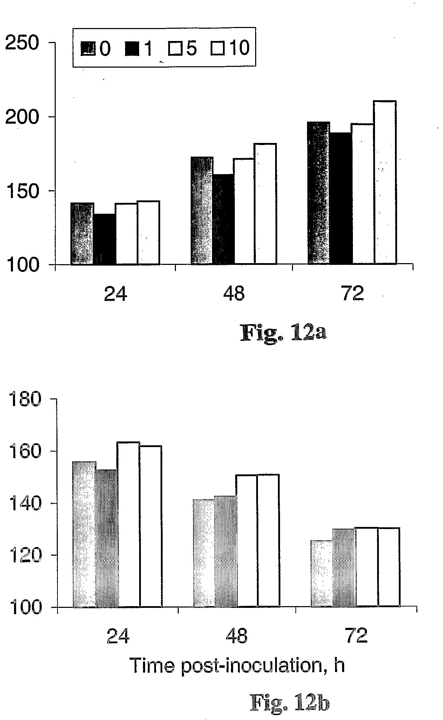

*Natural killer cell cytotoxic activity.* There were no dietary treatment differences in NK cell cytotoxic activity at week 8. However, astaxanthin stimulated NK cell cytotoxic activity on week 12 with effector:target ratios of 5:1 (5 and 10 mg astaxanthin) or 10:1 (1, 5 and 10 mg astaxanthin) (Figure 12).

Cats fed astaxanthin showed increased DTH response to both vaccine and con A. Dogs showed significant DTH response to vaccine. Heightened DTH response was accompanied by an increase in the populations of total T and Th lymphocytes in cats fed astaxanthin although no similar response was seen in dogs.

Mitogen-induced PBMC proliferation was generally enhanced in cats and dogs fed astaxanthin. In addition, NK cell cytotoxic activity was heightened in cats and dogs fed a diet composition containing astaxanthin.

Dietary astaxanthin also stimulated humoral immunity in both cats and dogs, with astaxanthin supplementation providing an increase in IgG and IgM production both before and after vaccination. Antibody production was generally heightened after antigen exposure through vaccination, as well. In contrast to dogs, enhanced antibody production in astaxanthin-supplemented cats was accompanied by a decrease in the B cell population when compared to un-supplemented cats. However, the populations of total T and Th cells in astaxanthin-fed cats were higher. It may be that astaxanthin increased antibody production in cats by stimulating T cell function, which would agree with the findings of a previous study in rodents that demonstrated increased T cell-mediated antibody production with astaxanthin supplementation (Jyonouchi *et al.*, 1994).

Enhancement of the immune functions of cats by astaxanthin may be attributed to the antioxidant property of astaxanthin, since the inventors did demonstrate that astaxanthin reduced lipid peroxidation in dogs. In summary, feeding astaxanthin to cats produced a dose-related increase in plasma astaxanthin. Concurrently, dietary astaxanthin heightened cell-mediated immune response as shown by enhanced DTH response to specific and nonspecific antigens, PBMC proliferation, NK cell cytotoxic activity, and increased population of total T and Th cells.

**References**

Bass et al., Am. J. Vet. Res., 37, 1355-1357 (1976).

Bennedsen et al., Immunology Lett., 70, 185-189 (1999).

Bertam, J. (1999) Carotenoids and gene regulation. Nutr. Rev. 57: 182-191.

Bjerkeng et al., Aquaculture, 157, 63-82 (1997).

Britton, G., FASEB J., 9, 1551-1558 (1995).

Brown et al., Am. J. Clin. Nutr., 49,1258-1265 (1989).

Chew et al., J. Anim. Sci., 69, 4892-4897 (1991).

Chew et al., J. Anim. Sci., 71, 730-739 (1993).

Cerveny et al., FASEB J., 13, A210 (1999).

Cerveny et al., FASEB J., 13, A210 (1999).

Chew, B. P., J. Nutr., 125, 1804S-1808S (1995a).

Chew, B. P., The influence of vitamins on reproduction in pigs. In: Recent Advances in Animal Nutrition. (P. C. Garnsworthy and D. J. A. Cole, eds.) pp 223-239. Nottingham Univ. Press, Nottingham, England (1995b).

Chew et al., Anticancer Res., 19, 1849-1853 (1999a).

Chew et al., Anticancer Res., 19, 5223-5227 (1999b).

Chew et al., J. Nutr., 130, 1788-1791 (2000a).

Chew et al., J. Nutr., 130, 2322-2325 (2000b).

Cornwell et al., J. Lipid Res., 3, 65-70 (1961).

Esterbauer et al., Ann. N.Y. Acad. Sci., 570, 254-267 (1989).

Goulinet and Chapman, Arterioscler, Thromb. Vasc. Biol., 17, 786-796 (1997).

Gugger et al., J. Nutr., 122, 115-119 (1992).

Julien et al., Circ. Res., 49, 248-254 (1981).

Jyonouchi et al., Nutr Cancer, 21, 47-58 (1994).

Jyounouchi et al., J. Nutr., 125, 2483-2492 (1995).

Jyonouchi et al., Nutr. Cancer, 23(2), 171-183 (1995).

Jyonouchi et al., Nutr. Cancer, 36, 59-65 (2000).

Kim et al., Vet. Immunol. Immunopath., 74, 315-327 (2000a).

Kim et al., Vet. Immunol. Immunopath., 74, 331-341 (2000b).

Krinski et al., Arch. Biochem. Biophys., 73, 233-246 (1958).

Kurashige et al., Physiol. Chem. Phys. Med. NMR, 22, 27-38 (1990).

Lawlor and O'Brien, Nutr. Res., 15, 1695-1704 (1995).

Martin et al., J. Prakt. Chem., 341, 302-308 (1991).

Mathews-Roth, M.M., Clin. Chem., 24, 700-701 (1978).

Miki, W., Pure Appl. Chem., 63, 141-146 (1991).

Miyamoto et al., J. Vet. Med. Sci., 57, 347-349 (1995).

Naguib, Y. M. A., J. Agric. Food Chem., 48, 1150-1154 (2000).

Nakano et al., Biochemica et Biophysica Acta, 1426, 119-125 (1999).

Nakano et al., J. Agric. Food Chem., 43, 1570-1573 (1995).

Olson, J. A., Pure Appl. Chem., 66, 1011-1016 (1994).

Osterlie et al., J. Nutr., 129, 391-398 (1999).

Palozza and Krinsky, Arch. Biochem. Biophys., 297, 184-187 (1992).

Park et al., J. Nutr., 128, 1802-1806 (1998).

Park et al., Nutr. Cancer, 33, 206-212 (1999).

Poor et al., J. Nutr., 122, 262-268 (1992).

Romanchik et al., J. Nutr., 125, 2610-2617 (1995).

SAS (1991) SAS/STAT User's Guide. SAS Institute Inc., Cary, NC.

Shigenaga et al., Proc. Natl. Acad. Sci., 91, 10771-10778 (1994).

Tanaka et al., Carcinogenesis, 15, 15-19 (1994).

Terao, J., Lipids, 24, 659-661 (1989).

Terpstra et al., Anal. Biochem., 111, 149-257 (1981).

Weisburger, J. H., Am. J. Clin. Nutr., 53, 226S-237S (1991).

**Claims**

1. A pet food composition comprising astaxanthin, for use in enhancing immunity, enhancing longevity, or combination thereof, in a companion animal.

2. A pet food composition according to claim 1 wherein the astaxanthin is less than 3%, preferably from 0.0001% to 2%, more preferably from 0.001% to 1%, most preferably from 0.001% to 0.5% by weight of the composition.

3. A pet food composition according to claim 1 or 2 wherein the companion animal is a domestic cat or dog.

4. A pet food composition according to any of the claims 1 to 3 wherein the companion animal composition is a cat or dog food composition.

5. A pet food composition according to any of the preceding claims wherein the pet food composition comprises from 20% to 50%, alternatively from 20% to a 40%, or alternatively from 20% to 35% crude protein by weight of food composition.

6. A pet food composition according any of the preceding claims wherein the pet food composition comprises from 5% to 40%, alternatively from 10% to 35% fat by weight of food composition.

7. A pet food composition according to any of the preceding claims wherein the pet food composition comprises at least a source of carbohydrate.

8. A pet food composition according to any of the preceding claims wherein the pet food composition comprises at least a fiber source.

9. A pet food composition according to any of the preceding claims wherein the pet food composition comprises probiotic microorganisms.

10. A pet food composition according to claim 1 wherein the pet food composition is a supplement.

11. A pet food composition comprising astaxanthin, for use in:

    increasing the cell-mediated immune response in a dog or cat;
    increasing the humoral immune response in a companion animal;
    increasing in vivo production of IgG and IgM in a companion animal; or
    attenuation of inflammation in a companion animal.

**Patentansprüche**

1. Tiernahrungszusammensetzung, die Astaxanthin umfasst, zur Verwendung in der Immunitätsstärkung, Erhöhung der Langlebigkeit oder Kombinationen aus diesen, bei einem Haustier.

2. Tiernahrungszusammensetzung nach Anspruch 1, wobei das Astaxanthin weniger als 3%, vorzugsweise 0,0001% bis 2%, bevorzugter 0,001% bis 1%, am bevorzugtesten 0,001% bis 0,5% auf das Gewicht der Zusammensetzung bezogen beträgt.

3. Tiernahrungszusammensetzung nach Anspruch 1 oder 2, wobei das Haustier eine Hauskatze oder ein Haushund ist.

4. Tiernahrungszusammensetzung nach einem der Ansprüche 1 bis 3, wobei es sich bei der Haustierzusammensetzung um eine Katzen- oder Hundefutterzusammensetzung handelt.

5. Tiernahrungszusammensetzung nach einem der vorhergehenden Ansprüche, wobei die Tiernahrungszusammensetzung 20% bis 50%, alternativ 20% bis 40% oder alternativ 20% bis 35% Rohprotein auf das Gewicht der Nahrungszusammensetzung bezogen umfasst.

6. Tiernahrungszusammensetzung nach einem der vorhergehenden Ansprüche, wobei die Tiernahrungszusammensetzung 5% bis 40%, alternativ 10% bis 35% Fett auf das Gewicht der Nahrungszusammensetzung bezogen umfasst.

7. Tiernahrungszusammensetzung nach einem der vorhergehenden Ansprüche, wobei die Tiernahrungszusammensetzung zumindest eine Kohlenhydratquelle umfasst.

8. Tiernahrungszusammensetzung nach einem der vorhergehenden Ansprüche, wobei die Tiernahrungszusammensetzung zumindest eine Ballaststoffquelle umfasst.

9. Tiernahrungszusammensetzung nach einem der vorhergehenden Ansprüche, wobei die Tiernahrungszusammensetzung probiotische Mikroorganismen umfasst.

10. Tiernahrungszusammensetzung nach Anspruch 1, wobei es sich bei der Tiernahrungszusammensetzung um ein Ergänzungsmittel handelt.

11. Tiernahrungszusammensetzung, die Astaxanthin umfasst, zur Verwendung beim:

Steigern der zellvermittelten Immunabwehr bei einem Hund oder einer Katze;
Steigern der humoralen Immunabwehr bei einem Haustier;
Steigern der *in vivo*-Produktion von IgG und IgM bei einem Haustier; oder
Abschwächen einer Entzündung bei einem Haustier.

**Revendications**

1. Composition alimentaire pour animal de compagnie comprenant de l'astaxanthine, destinée à être utilisée pour l'augmentation de l'immunité, l'augmentation de la longévité, ou une combinaison de celles-ci, chez un animal de compagnie.

2. Composition alimentaire pour animal de compagnie selon la revendication 1, sachant que l'astaxanthine est inférieure à 3%, de préférence de 0,0001 % à 2%, de façon plus préférentielle de 0,001% à 1%, de façon la plus préférentielle de 0,001% à 0,5% en poids de la composition.

3. Composition alimentaire pour animal de compagnie selon la revendication 1 ou 2, sachant que l'animal de compagnie est un chat ou un chien domestique.

4. Composition alimentaire pour animal de compagnie selon l'une quelconque des revendications 1 à 3, sachant que la composition pour animal de compagnie est une composition alimentaire pour chat ou chien.

5. Composition alimentaire pour animal de compagnie selon l'une quelconque des revendications précédentes, sachant que la composition alimentaire pour animal de compagnie comprend de 20% à 50%, alternativement de 20% à 40%, ou alternativement de 20% à 35% de protéine brute en poids de la composition alimentaire.

6. Composition alimentaire pour animal de compagnie selon l'une quelconque des revendications précédentes, sachant que la composition pour animal de compagnie comprend de 5% à 40%, alternativement de 10% à 35% de graisse en poids de la composition alimentaire.

7. Composition alimentaire pour animal de compagnie selon l'une quelconque des revendications précédentes, sachant que la composition pour animal de compagnie comprend au moins une source de carbohydrate.

8. Composition alimentaire pour animal de compagnie selon l'une quelconque des revendications précédentes, sachant que la composition pour animal de compagnie comprend au moins une source de fibres.

9. Composition alimentaire pour animal de compagnie selon l'une quelconque des revendications précédentes, sachant que la composition pour animal de compagnie comprend des micro-organismes probiotiques.

10. Composition alimentaire pour animal de compagnie selon la revendication 1, sachant que la composition pour animal de compagnie est un supplément.

11. Composition alimentaire pour animal de compagnie comprenant de l'astaxanthine, destinée à être utilisée pour :

l'augmentation de la réponse immunitaire à médiation cellulaire chez un chien ou un chat ;
l'augmentation de la réponse immunitaire humorale chez un animal de compagnie ;
l'augmentation de production *in vivo* d'IgG et d'IgM chez un animal de compagnie ; ou
l'atténuation d'une inflammation chez un animal de compagnie.

Fig. 1

## Fig. 2

**Fig. 3**

## Fig. 4

## Fig. 5a

Period Post- injection, h

## Fig. 5b

# Fig. 6

**Fig. 7a**

**Fig. 7b**

**Fig. 8**

Fig. 9.

# Fig. 10

# Fig. 11

**Fig. 12a**

Time post-inoculation, h

**Fig. 12b**

**Fig. 13a**

**Fig. 13b**

Period of feeding, wk

**Fig. 13c**

**Fig. 14a**

**Fig. 14b**

Period of feeding, wk

**Fig. 14c**

Fig. 15a

Fig. 15b

Fig. 16a

Fig. 16b

Period of feeding, wk

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6310090 B1
- WO 9735491 A
- US 5744502 A

### Non-patent literature cited in the description

- **BASS et al.** *Am. J. Vet. Res.,* 1976, vol. 37, 1355-1357
- **BENNEDSEN et al.** *Immunology Lett.,* 1999, vol. 70, 185-189
- **BERTAM, J.** Carotenoids and gene regulation. *Nutr. Rev.,* 1999, vol. 57, 182-191
- **BJERKENG et al.** *Aquaculture,* 1997, vol. 157, 63-82
- **BRITTON, G.** *FASEB J.,* 1995, vol. 9, 1551-1558
- **BROWN et al.** *Am. J. Clin. Nutr.,* 1989, vol. 49, 1258-1265
- **CHEW et al.** *J. Anim. Sci.,* 1991, vol. 69, 4892-4897
- **CHEW et al.** *J. Anim. Sci.,* 1993, vol. 71, 730-739
- **CERVENY et al.** *FASEB J.,* 1999, vol. 13, A210
- **CHEW, B. P.** *J. Nutr.,* 1995, vol. 125, 1804S-1808S
- The influence of vitamins on reproduction in pigs. **CHEW, B. P.** Recent Advances in Animal Nutrition. Nottingham Univ. Press, 1995, 223-239
- **CHEW et al.** *Anticancer Res.,* 1999, vol. 19, 1849-1853
- **CHEW et al.** *Anticancer Res.,* 1999, vol. 19, 5223-5227
- **CHEW et al.** *J. Nutr.,* 2000, vol. 130, 1788-1791
- **CHEW et al.** *J. Nutr.,* 2000, vol. 130, 2322-2325
- **CORNWELL et al.** *J. Lipid Res.,* 1961, vol. 3, 65-70
- **ESTERBAUER et al.** *Ann. N.Y. Acad. Sci.,* 1989, vol. 570, 254-267
- **GOULINET ; CHAPMAN.** *Arterioscler, Thromb. Vasc. Biol.,* 1997, vol. 17, 786-796
- **GUGGER et al.** *J. Nutr.,* 1992, vol. 122, 115-119
- **JULIEN et al.** *Circ. Res.,* 1981, vol. 49, 248-254
- **JYONOUCHI et al.** *Nutr Cancer,* 1994, vol. 21, 47-58
- **JYOUNOUCHI et al.** *J. Nutr.,* 1995, vol. 125, 2483-2492
- **JYONOUCHI et al.** *Nutr. Cancer,* 1995, vol. 23 (2), 171-183
- **JYONOUCHI et al.** *Nutr. Cancer,* 2000, vol. 36, 59-65
- **KIM et al.** *Vet. Immunol. Immunopath.,* 2000, vol. 74, 315-327
- **KIM et al.** *Vet. Immunol. Immunopath.,* 2000, vol. 74, 331-341
- **KRINSKI et al.** *Arch. Biochem. Biophys.,* 1958, vol. 73, 233-246
- **KURASHIGE et al.** *Physiol. Chem. Phys. Med. NMR,* 1990, vol. 22, 27-38
- **LAWLOR ; O'BRIEN.** *Nutr. Res.,* 1995, vol. 15, 1695-1704
- **MARTIN et al.** *J. Prakt. Chem.,* 1991, vol. 341, 302-308
- **MATHEWS-ROTH, M.M.** *Clin. Chem.,* 1978, vol. 24, 700-701
- **MIKI, W.** *Pure Appl. Chem.,* 1991, vol. 63, 141-146
- **MIYAMOTO et al.** *J. Vet. Med. Sci.,* 1995, vol. 57, 347-349
- **NAGUIB, Y. M. A.** *J. Agric. Food Chem.,* 2000, vol. 48, 1150-1154
- **NAKANO et al.** *Biochemica et Biophysica Acta,* 1999, vol. 1426, 119-125
- **NAKANO et al.** *J. Agric. Food Chem.,* 1995, vol. 43, 1570-1573
- **OLSON, J. A.** *Pure Appl. Chem.,* 1994, vol. 66, 1011-1016
- **OSTERLIE et al.** *J. Nutr.,* 1999, vol. 129, 391-398
- **PALOZZA ; KRINSKY.** *Arch. Biochem. Biophys.,* 1992, vol. 297, 184-187
- **PARK et al.** *J. Nutr.,* 1998, vol. 128, 1802-1806
- **PARK et al.** *Nutr. Cancer,* 1999, vol. 33, 206-212
- **POOR et al.** *J. Nutr.,* 1992, vol. 122, 262-268
- **ROMANCHIK et al.** *J. Nutr.,* 1995, vol. 125, 2610-2617

- **SAS.** SAS/STAT User's Guide. SAS Institute Inc, 1991
- **SHIGENAGA et al.** *Proc. Natl. Acad. Sci.,* 1994, vol. 91, 10771-10778
- **TANAKA et al.** *Carcinogenesis,* 1994, vol. 15, 15-19
- **TERAO, J.** *Lipids,* 1989, vol. 24, 659-661
- **TERPSTRA et al.** *Anal. Biochem.,* 1981, vol. 111, 149-257
- **WEISBURGER, J. H.** *Am. J. Clin. Nutr.,* 1991, vol. 53, 226S-237S